# EUROPÄISCHE PATENTSCHRIFT

(11) **EP 1 166 751 B1**
(45) Veröffentlichungstag und Bekanntmachung des Hinweises auf die Patenterteilung: **15.09.2004**
(21) Anmeldenummer: 01113596.9
(22) Anmeldetag: 15.06.2001
(51) Int. Cl.: A61K 7/13

(54) **Mittel zum Färben von menschlichen Haaren**
Dyeing composition for human hair
Composition de teinture pour cheveux humains

(30) Priorität: 19.06.2000 DE 10030097
(43) Veröffentlichungstag der Anmeldung: 02.01.2002
(73) Patentinhaber: KPSS-Kao Professional Salon Services GmbH, 64297 Darmstadt (DE)
(72) Erfinder: Nöcker, Bernd, Dr., 64372 Ober-Ramstadt (DE); Theis, Heinz, Dr., 64354 Reinheim (DE); Kure, Naohisa, Dr., Sumida-ku, Tokyo 131 (JP)

(56) Entgegenhaltungen:
- EP-A- 0 640 643
- DE-C- 19 718 185
- US-A- 5 143 518
- DATABASE WPI Section Ch, Week 198532 Derwent Publications Ltd., London, GB; Class A26, AN 1985-192980 XP002258901 & JP 60 119279 A (SUNSTAR KK) 26. Juni 1985 (1985-06-26)

## Beschreibung

Die vorliegende Erfindung betrifft ein Mittel zum Färben von menschlichen Haaren auf der Basis von Oxidationsfarbstoffen, das eine ausgezeichnete Farbwirkung besitzt, insbesondere eine gleichmäßige Färbung mit guter Stabilität ergibt, auf die Haarstruktur auch bei mehrfacher Anwendung keinerlei schädigende, sondern vielmehr eine konditionierende Wirkung ausübt und einen ausdrucksvollen Farbglanz hervorruft.

Die Oxidations-Haarfärbung erfordert bekanntlich das vorherige Mischen von zwei bis zur Anwendung getrennt gehaltenen Zusammensetzungen aus Oxidationsfarbstoff-Vorprodukt und Oxidationsmittel und das anschließende Aufbringen der erhaltenen Mischung.

Diese Zusammensetzungen werden üblicherweise bei einem pH-Wert zwischen etwa 8 und 11, insbesondere 9 bis 10,5, eingesetzt, was bei wiederholter, zeitlich nahe zusammenliegender Anwendung zu Haarschädigungen führen kann.
Außerdem ist die Farbwirkung, insbesondere die Farbstabilität und Gleichmäßigkeit der Färbung, noch verbesserungsfähig.

Es wurde nunmehr gefunden, daß diese Nachteile überwunden werden können und ein Haarfärbemittel auf Basis mindestens eines Oxidationsfarbstoff-Vorprodukts, vorzugsweise eines Entwickler-Kuppler-Systems, das eine gleichmäßige Farbwirkung besitzt und die Haarstruktur in keiner Weise schädigt, sondern dem gefärbten Haar Glanz und eine gute Kämmbarkeit verleiht, dadurch hergestellt werden kann, wenn man diesem Mittel mindestens ein Organopolysiloxan zusetzt, bei dem mindestens ein Siliciumatom über eine Alkylengruppe, die ein Heteroatom, insbesondere ein Stickstoffatom, aufweist, mit einem Poly-(N-acylalkylenimin) mit Einheiten der Formel I 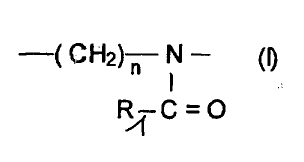 wobei n eine Zahl von 1 bis 5 und R₁ Wasserstoff, eine C₁-C₁₂-Alkyl- oder-Cycloalkyl-, -Aralkyl- oder -Arylgruppe bedeuten, verbunden ist, vorzugsweise in einer Menge von 0,01 bis 5 Gew.-%, insbesondere 0,05 bis 2,5 Gew.-%, bezogen auf die Gesamtmenge des Mittels.

Bevorzugte Organopolysiloxan-Polymere sind solche, wie sie in der EP-A 640 643 beschrieben sind, insbesondere gegebenenfalls quaternierte Aminoalkyl-, insbesondere Aminopropyldimethylpolysiloxan/Polyethyloxazolin-Copolymerisate der Formel 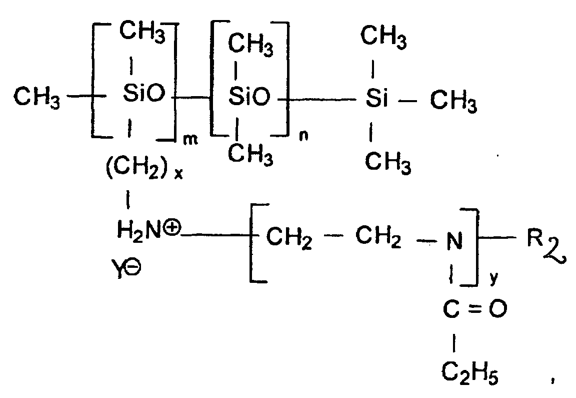 worin m und n jeweils ganze Zahlen von 20 bis 10 000, insbesondere 50 bis 7000, vor allem 100 bis 5000, x eine Zahl zwischen 1 und 5, vorzugsweise 3, und y eine Zahl von 5 bis 30 bedeuten, R₂ eine C₁-C₁₂-Alkyl- oder Arylgruppe, insbesondere eine Methyl-, Ethyl- oder Benzylgruppe, und Y⁻ ein Anion darstellen.

Die Herstellung der erfindungsgemäß bevorzugt zum Einsatz gelangenden Aminoalkyldimethylpolysiloxan/Polyethyloxazolin-Pfropfcopolymerisate ist in der bereits erwähnten EP-A 640 643 beschrieben und erfolgt entsprechend dem folgenden Schema:

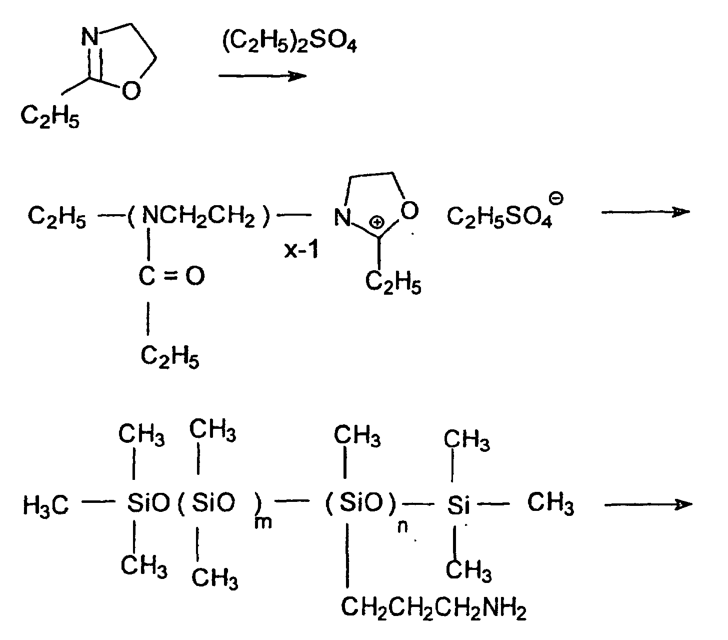

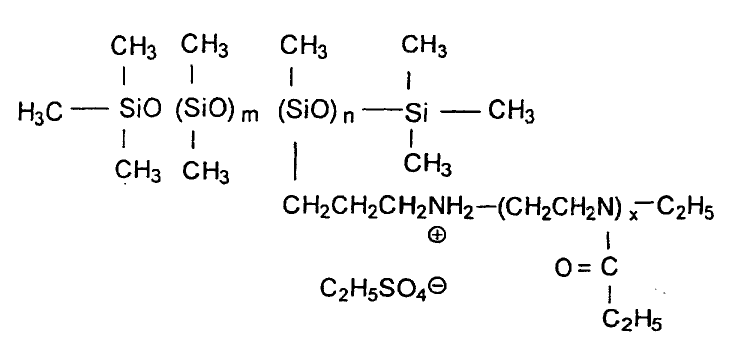

Das Anion Y⁻ der allgemeinen Formel kann selbstverständlich auch ein anderes als das Ethylsulfat-Anion des obengenannten Beispiels sein, d.h. die Quaternierung kann auch mit Methylchlorid, Dimethylsulfat, Benzylchlorid, Dodecylbromid etc. erfolgen.

Ein besonders bevorzugtes Pfropfcopolymerisat der dargestellten Art weist ein Gesamtmolekulargewicht von etwa 50 000 bis etwa 500 000, vorzugsweise etwa 80 000 bis etwa 300 000, insbesondere etwa 100 000 Dalton auf, wobei das Molgewicht des Oxazolin-Segments etwa 2 500 bis etwa 7 500, vorzugsweise etwa 4 000 bis etwa 6 000, insbesondere etwa 5 000 Dalton/Segment beträgt, d.h. der Molanteil bei 20 Einheiten/Molekül liegt. Der bevorzugte Si-Gehalt beträgt, entsprechend der Elementaranalyse, etwa 50 %.

Besonders geeignet sind die unter den Bezeichnungen A-1, A-2 und A-3 auf den Seiten 12 bis 13 der EP-A 640 643 beschriebenen Organopolysiloxane. Der Anteil der Pfropfcopolymerisate in den erfindungsgemäßen Haarfärbemitteln liegt bei etwa 0,05 bis 5, vorzugsweise 0,1 bis 2,5, insbesondere 0,5 bis 1,5 Gew.-% der Gesamtzusammensetzung.

Die erfindungsgemäß zugesetzten Polymeren können vorzugsweise der das Oxidationsfarbstoff-Vorprodukt enthaltenden Zusammensetzung zugesetzt werden; es ist jedoch auch möglich, sie in der Oxidationsmittel-Zusammensetzung oder in beiden einzusetzen.

Diese Zusammensetzung sind per se aus dem Stand der Technik bekannt und beispielsweise in der Monographie von K. Schrader, Grundlagen und Rezepturen der Kosmetika, 2. Aufl. (Hüthig Buchverlag, 1989), S. 782-804, beschrieben.

Wie bereits angedeutet, liegt der pH-Wert der gebrauchsfertigen Zusammensetzung vorzugsweise im alkalischen Bereich zwischen etwa 8 und 11, insbesondere etwa 9 bis 10,5, er kann jedoch auch im schwach sauren (etwa 5 bis 6,9, insbesondere etwa 5,5 bis 6,5) oder neutralen Bereich liegen.

Die möglicherweise etwa schwächere Färbeintensität im sauren Bereich wird dabei durch den erfindungsgemäßen Polymerzusatz wesentlich erhöht.

Wie bereits gesagt, können grundsätzlich alle aus dem Stand der Technik für die Haarfärbung bekannten Entwickler- und Kupplersubstanzen eingesetzt werden.

Beispielhafte Entwicklersubstanzen sind insbesondere 1,4-Diaminobenzol, 2,5-Diaminotoluol, Tetraaminopyrimidine, Triaminohydroxypyrimidine, 1,2,4-Triaminobenzol, 2-(2,5-Diaminophenyl)ethanol, 4 Aminophenol, 4-Amino-3-methylphenol, 2-(2-Hydroxyethylamino)-5-aminotoluol und 1-Amino-4-bis-(2'-hydroxyethyl)-aminobenzol bzw. deren wasserlöslichen Salze; beispielhafte Kupplersubstanzen sind Resorcin, 2-Methylresorcin, 4-Chlorresorcin, 2-Amino-4-chlorphenol, 4-(N-methyl)aminophenol, 2-Aminophenol, 3-Aminophenol, 1-Methyl-2-hydroxy-4-aminobenzol, 3-N,N-Dimethylaminophenol, 4-Amino-3-methylphenol, 5-Amino-2-methylphenol, 6-Amino-3-methylphenol, 3-Amino-2-methylamino-6-methoxypyridin, 2-Amino-3-hydroxypyridin, 4-Aminodiphenylamin, 4,4'-Diaminodophenylamin, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'-hydroxyethyl)amino]benzol, α-Naphthol, 1,4-Diamino-2-chlor-benzol, 4,6-Dichlorresorcin, 1,3-Diaminotoluol, 4-Hydroxy-1,2-methylendioxybenzol, 1,5-Dihydroxynaphthalin, 1,6-Dihydroxynaphthalin, 1,7-Dihydroxynaphthalin, 2,7-Dihydroxynaphthalin, 1-Hydroxynaphthalin, 4-Hydroxy-1,2-Methylendioxybenzol, 2,4-Diamino-3-chlorphenol und/oder 1-Methoxy-2-amino-4-(2'-hydroxyethylamino)benzol, ohne daß diese beispielhafte Aufzählung Anspruch auf Vollständigkeit erheben könnte.

Entwickler- und Kupplersubstanzen sind vorzugsweise im Molverhältnis 1:3 bis 5:1, insbesondere etwa 1:1 und etwa 3:1, enthalten; ihr Anteil in den erfindungsgemäß eingesetzten Farbmischungen kann jeweils etwa 0,25 bis etwa 5 Gew.-%, je nach gewünschter Färbung betragen.

Als Oxidationsmittel werden vor allem verdünnte Wasserstoffperoxid-Lösungen, -Emulsionen oder -Gele eingesetzt, möglich, aber weniger üblich ist auch die Verwendung weiterer Peroxide wie Erdalkaliperoxiden, Harnstoffperoxid, Melaminperoxid, etc. in entsprechenden stöchiometrischen Mengen.

Die Oxidationsfarbstoffzusammensetzungen können als Lösungen, Cremes, Pasten, Gele, Aerosole, etc. Verwendung finden.

Die Einwirkungszeit auf dem Haar liegt vorzugsweise bei jeweils etwa fünf bis dreißig Minuten, insbesondere bei jeweils etwa 10 bis 20, beispielsweise jeweils 15 Minuten für die alkalische und angesäuerte Farbmischung. Das erfindungsgemäße Verfahren eignet sich dabei sowohl zum ganzheitlichen, d.h. erstmaligen Färben der Haare, als auch zum Nachfärben.

Die erfindungsgemäß eingesetzten Mittel können selbstverständlich alle in Haarfärbemitteln üblichen Stoffe enthalten, auf deren detaillierte Aufzählung hier verzichtet wird, und als (wäßrige) Lösungen, Emulsionen, Cremes, Schäume etc. vorliegen.

Auch die Mitverwendung direktziehender Haarfarbstoffe zur Nuancierung der erwünschten Farbtöne ist möglich.

Zur Vermeidung von Wiederholungen wird hierzu auf den Stand der Technik verwiesen, wie er beispielsweise in der Monographie von K. Schrader, l.c. beschrieben ist.

Die dort geoffenbarten Zusammensetzungen und Einzelbestandteile, auf die ausdrücklich Bezug genommen wird, können auch im Rahmen der vorliegenden Erfindung verwendet werden.

Die folgenden Beispiele dienen der Illustration der Erfindung.

### Beispiel 1

Auf Strähnen gebleichten Menschenhaares wurde eine Färbezusammensetzung, die durch Vermischen von gleichen Gewichtsteilen einer 6%-igen Wasserstoffperoxidlösung und eines Oxidationsfarbstoffvorprodukts der folgenden Zusammensetzung erhalten wurde, und einen pH-Wert von 9,8 aufwies, aufgebracht.

| **Trägermasse** | |
|---|---|
| Cetylstearylalkohol | 11,00 (Gew.-%) |
| Oleth-5 | 5,00 |
| Ölsäure | 2,50 |
| Stearinsäuremonoethanolamid | 2,50 |
| Cocosfettsäuremonoethanolamid | 2,50 |
| Natriumlaurylsulfat | 1,70 |
| Natriumsulfit | 1,00 |
| 1,2-Propandiol | 1,00 |
| Ascorbinsäure | 0,50 |
| Ammoniumchlorid | 0,50 |
| EDTA, Tetranatriumsalz | 0,20 |
| Parfum | 0,40 |
| Weizenproteinhydrolysat | 0,20 |
| Silica | 0,10 |
| Alkyldimethylpolysiloxan/Polyé-ethyloxazolin-Pfropfcopolymerisat (Organopolysiloxan A-1 der EP-A 640 643) | 0,40 |

| Oxidationsfarbstoffmischung | |
|---|---|
| 2,5,6-Triamino-4-hydroxypyrimidinsulfat | 0,01 |
| 2,5-Diaminotoluolsulfat | 0,55 |
| 4-Chlorresorcin | 0,17 |
| Resorcin | 0,05 |
| 3-Aminophenol | 0,03 |
| Ammoniak | q.s. |
| Wasser | ad 100,0 |

Nach zwanzigminütiger Einwirkung wurde gewaschen, gespült und getrocknet.

Es wurde eine glänzende, intensive, gleichmäßige Mittelblondfärbung erhalten.

Das Weglassen des Pforpfcopolymerisats führte zu einer deutlich weniger intensiven und gleichmäßigen Färbung mit verringertem Glanz.

Darüberhinaus waren sowohl die Naß- als auch die Trockenkämmbarkeit sowie der Griff des Haares deutlich schlechter.

### Beispiel 2

In eine der Zusammensetzung nach Beispiel 1 entsprechende Trägermasse , die jedoch als Polymer nur 0,15 Gew.-% eines Organopolysiloxans nach Beispiel A-2 der EP-A 640 643 enthielt, wurde die folgende Oxidationsfarbstoffmischung eingebracht:

| | |
|---|---|
| 2,5,6-Triamino-4-hydroxypyrimidinsulfat | 0,01 (Gew.-%) |
| 2,5-Diaminotoluolsulfat | 0,90 |
| 2-Amino-4-hydroxypyridin | 0,80 |
| 1-Naphthol | 0,17 |
| 3-Aminophenol | 0,10 |
| Resorcin | 0,03 |

Diese Produkt wurde mit 6%-iger H₂O₂-Lösung im Gewichtsverhältnis 1:1 vermischt (pH 9,5) und auf Strähnen aus gebleichtem Menschenhaar aufgebracht.

Nach 20-minütiger Einwirkung wurde mit Wasser ausgewaschen, shampooniert und getrocknet.

Es wurde eine intensive, glänzende, gleichmäßige Mahagoni-Färbung erhalten.

Nach Anwendung einer Zusammensetzung ohne das Polymer wies das Haar nicht nur eine weniger intensive und gleichmäßige Färbung, sondern auch einen rauhen Griff auf.

### Beispiel 3

Die in Beispiel 1 beschriebene Zusammensetzung wurde dahingehend variiert, daß sie 1,0 Gew.-% Organopolysiloxan Polymer A1 nach der EP-A 640 643 enthielt und so eingestellt war, daß beim Vermischen mit 6%-iger H₂O₂-Lösung ein pH-Wert von 6,5 erreicht wurde.
Die Färbung wurde, wie beschrieben, durchgeführt.
Es wurde wiederum eine gleichmäßige, intensive, glänzende Blondfärbung erhalten.
Weglassen des Polymers resultierte nicht nur in verschlechterten Haareigenschaften wie rauherer Griff, Naß- und Trockenkämmbarkeit sowie geringere Spannkraft, sondern vor allem auch in deutlich reduzierter Farbintensität, Gleichmäßigkeit der Färbung und weniger Glanz.

### Beispiel 4

In eine der Zusammensetzung nach Beispiel 1 entsprechende Trägermasse, die jedoch kein Organopolysiloxan enthielt, wurde die folgende Oxidationsfarbstoffmischung eingebracht:

| | |
|---|---|
| 2,5-Diaminotoluolsulfat | 0,80 (Gew.-%) |
| Resorcin | 0,30 |
| 2-Amino-4-hydroxypyridin | 0,06 |
| 3-Aminophenol | 0,03 |

Dieses Produkt wurde mit 2%-iger H₂O₂-Lotion im Gewichtsverhältnis 1:2 gemischt (pH 9,6) und auf Strähnen aus gebleichtem Menschenhaar aufgebracht.

Nach 20-minütiger Einwirkung wurde gründlich ausgespült und getrocknet.

Es wurde eine glänzende, gleichmäßige intensive rotbraune Färbung erhalten, die auch nach fünf Haarwäschen noch stabil war, wobei das Haar einen weichen, lockeren Griff aufwies.

| | |
|---|---|
| **Zusammensetzung der H**_{**2**}**O**_{**2**}**-Lotion:** | |
| Dinatriumhydrogenphosphat | 0,1 (Gew.-%) |
| Etidronsäure | 0,3 |
| Triethanolaminlaurylsulfat | 0,7 |
| Poloxamer^{R}407 | 1,0 |
| PEG-7-Glycerylcocat | 0,5 |
| Wasserstoffperoxid | 2,0 |
| Alkyldimethylsiloxan/Polyethyloxazolinpfropfcopolymerisat (Organopolysiloxan A-1 der EP-A 640 643) | 1,5 |
| Wasser | ad 100,0 |

Verwendung der H₂O₂-Lotion ohne das Organopolysiloxan-Polymer, ersetzt durch die gleiche Menge eines üblichen Polymeren, Polyquaternium-7, erbrachte eine deutliche reduzierte Intensität und Gleichmäßigkeit sowie verringerten Glanz der Färbung.

## Patentansprüche

1. Mittel zum Färben von menschlichen Haaren, enthaltend
a) mindestens ein Oxidationsfarbstoff-Vorprodukt, und
b) mindestens ein Organopolysiloxan, bei dem mindestens ein Siliciumatom über eine Alkylengruppe, die ein Heteroatom, insbesondere ein Stickstoffatom, aufweist, mit einem Poly-(N-acylalkylenimin) mit Einheiten der Formel I 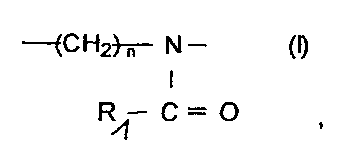
wobei n eine Zahl von 1 bis 5 und R₁ Wasserstoff, eine C₁-C₁₂-Alkyl-, Cycloalkyl-, Aralkyl- oder Arylgruppe bedeuten, verbunden ist.

2. Mittel nach Anspruch 1, enthaltend als Organosiloxan ein gegebenenfalls quaterniertes Aminoalkyldimethylpolysiloxan/Polyethyloxazolin-Copolymerisat der Formel 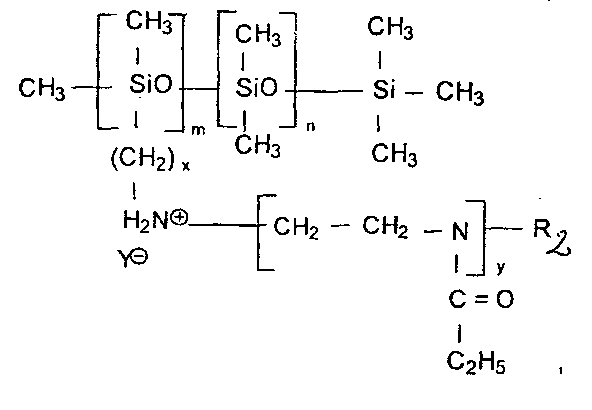 worin m und n jeweils ganze Zahlen von 20 bis 10 000, insbesondere 50 bis 7000, vor allem 100 bis 5000, x eine Zahl zwischen 1 und 5, vorzugsweise 3, und y eine Zahl von 5 bis 30 bedeuten, R₂ eine C₁-C₁₂-Alkyl- oder Arylgruppe, insbesondere eine Methyl-, Ethyl- oder Benzylgruppe, und Y⁻ ein Anion darstellen.

3. Mittel nach Anspruch 1 oder 2, enthaltend 0,05 bis 5 Gew.-%, berechnet auf die Gesamtzusammensetzung, des Organopolysiloxans.

4. Mittel nach Anspruch 3, enthaltend 0,1 bis 2,5 Gew.-% des Organopolysiloxans, berechnet auf die Gesamtzusammensetzung des Mittels.

5. Verwendung eines Organopolysiloxans gemäß Anspruch 1 in Mitteln zum Färben von menschlichen Haaren, die mindestens ein Oxidationsfarbstoff-Vorprodukt enthalten.

## Claims

1. Composition for the dyeing of human hair, comprising
a) at least one oxidation dyestuff precursor, and
b) at least one organopolysiloxane, wherein at least one silicium atom is connected by an alkylene group having a hetero-atom, in particular a nitrogen atom, to a poly-(N-acyl alkyleneimine) with units of the formula I, 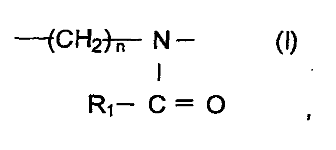
wherein n is a number from 1 to 5 and R₁ is hydrogen, a C₁-C₁₂-alkyl, cycloalkyl-, aralkyl- or aryl group.

2. Composition according to claim 1, containing as organosiloxane an optionally quaternized aminoalkyl dimethyl polysiloxane/polyethyl oxazoline copolymer of the formula 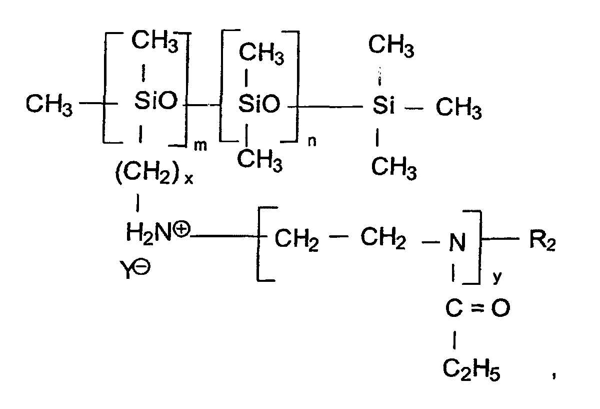 wherein m and n are each numbers from 20 to 10,000, in particular 50 to 7,000, especially 100 to 5,000, x is a number between 1 and 5, preferably 3, and y is a number from 5 to 30, R₂ is a C₁-C₁₂-alkyl or aryl group, in particular a methyl, ethyl or benzyl group, and Y⁻ is an anion.

3. Composition according to claim 1 or 2, containing 0.05 % to 5 % by weight, calculated to the total composition, of the organopolysiloxane.

4. Composition according to claim 3, containing 0.1 % to 2.5 % by weight, calculated to the total composition, of the organopolysiloxane.

5. Use of an organopolysiloxane according to claim 1 in compositions for the dyeing of human hair comprising at least one oxidation dyestuff precursor.

## Revendications

1. Produit de coloration des cheveux humains, contenant
a) au moins un précurseur de colorant d'oxydation, et
b) au moins un organopolysiloxane, pour lequel au moins un atome de silicium est relié, par l'intermédiaire d'un groupe alkyle, qui présente un hétéroatome, en particulier un atome d'azote, à une poly-(N-acylalkylène-imine) ayant des unités de formule I : 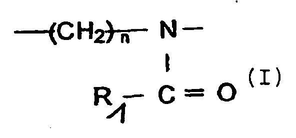
où n est un nombre allant de 1 à 5, et R₁ est l'hydrogène, un groupe alkyle en C₁-C₁₂, cycloalkyle, aralkyle, ou aryle.

2. Produit selon la revendication 1, contenant, en tant qu'organosiloxane, un copolymérisat, le cas échéant quaternisé, d'aminoalkyldiméthylpolysiloxane/polyéthyloxazoline de formule : 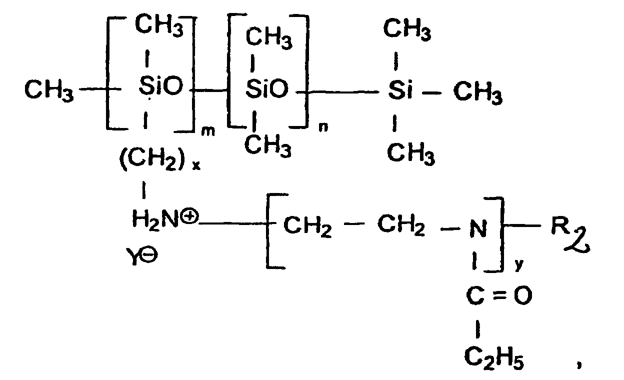 dans laquelle m et n sont chacun des nombres entiers allant de 20 à 10 000, en particulier de 50 à 7 000, surtout de 100 à 5 000, x est un nombre compris entre 1 et 5, de préférence 3, et y est un nombre allant de 5 à 30, R₂ est un groupe alkyle en C₁ à C₁₂ ou aryle, en particulier un groupe méthyle, éthyle ou benzyle, et Y⁻ est un anion.

3. Produit selon la revendication 1 ou 2, contenant de 0,05 à 5 % en poids, calculé sur la composition globale, de l'organopolysiloxane.

4. Produit selon la revendication 3, contenant de 0,1 à 2,5 % en poids, de l'organopolysiloxane, calculé sur la composition globale.

5. Utilisation d'un organopolysiloxane selon la revendication 1, dans des produits de coloration des cheveux humains, contenant au moins un précurseur de colorant d'oxydation.
